# EUROPÄISCHE PATENTANMELDUNG

(11) **EP 0 949 337 A2**
(43) Veröffentlichungstag der Anmeldung: **13.10.1999**
(21) Anmeldenummer: 99105198.8
(22) Anmeldetag: 01.04.1999
(51) Int. Cl.: C12Q 1/68

(54) **"Nachweisverfahren für Phytophthora-arten"**

(30) Priorität: 03.04.1998 DE 19815138
(71) Anmelder: GSF GESELLSCHAFT FÜR STRAHLEN- UND UMWELTFORSCHUNG MBH, D-85764 Oberschleissheim (DE)
(72) Erfinder: Bahnweg, Günther, 85368 Moosburg (DE); Schubert, Roland, 80993 München (DE); Sandermann, Heinrich, 81377 München (DE); Müller-Starck, Gerhard, 85354 Freising (DE)
(74) Vertreter: Behnisch, Werner, Dr.

(57) **Zusammenfassung**

Die Anmeldung betrifft ein Verfahren zum qualitativen und quantitativen Nachweis von Phytophthora citricola, Phytophthora cambivora und Phytophthora quercina, Primer zum artspezifischen Nachweis dieser Phytophthora-Arten sowie eine Verwendung der Primer-Oligonukleotide zum spezifischen Nachweis dieser Phytophthora-Arten.

## Beschreibung

Die Anmeldung betrifft ein Verfahren zum qualitativen und quantitativen Nachweis von Phytophthora citricola, Phytophthora cambivora und Phytophthora quercina, Primer zum artspezifischen Nachweis dieser Phytophthora-Arten sowie eine Verwendung der Primer-Oligonukleotide zum spezifischen Nachweis dieser Phytophthora-Arten

In den 60er Jahren dieses Jahrhunderts wurde in Osteuropa ein großflächiges Eichensterben beobachtet, welches in den 80er Jahren auch den Mittelmeerraum und Zentraleuropa erfaßte (zusammenfassend dargestellt von SIWECKI und LIESE, 1991). Das komplexe Schadbild ist u. a. durch Absterbeerscheinungen im Kronenraum, Blattvergilbungen und einen signifikanten Verlust an lebenden Feinwurzeln gekennzeichnet. In den 90er Jahren wurden aus in dieser Weise geschädigten Eichen- sowie Buchenbeständen einzelne Phytophthora-Arten isoliert, was zur Folge hatte, daß man in solchen Pathogenen einen primären Krankheitsverursacher beim Waldsterben vermutete (BRASIER, 1993; BLASCHKE, 1994; JUNG et al., 1996).

Zur Gattung Phytophthora gehören mehr als 60 einzelne Arten, und bei den meisten handelt es sich um weltweit verbreitete bodenbürtige Wurzelpathogene, welche die verschiedensten Kulturpflanzen, Strauch- und Baumspecies schädigen (zusammenfassende Darstellung siehe ERWIN and RIBEIRO, 1996). Phytophthora-Pathogene haben in der Geschichte der menschlichen Land- und Forstwirtschaft wiederholt Hungersnöte und enorme wirtschaftliche Verluste hervorgerufen.

Phytophthora-Organismen werden in klassischer Weise anhand selektiver Kulturverfahren nachgewiesen, wobei die Artunterscheidung auf der Grundlage morphologischer, physiologischer und biochemischer Marker beruht (NEWHOOK et al., 1978; STAMPS et al., 1990). Diese Untersuchungs-verfahren sind zeitaufwendig und setzen ein hohes mikrobiologisches Spezialwissen voraus (siehe TSAO, 1983). Darüber hinaus werden sie durch mögliche Kontaminationen mit anderen Mikroorganismen bzw. durch saisonbedingte Ruhephasen der Phytophthora-Pathogene in ihrem Erfolg beeinflußt. Man hat in den vergangenen Jahren deshalb zahlreiche serologische Testverfahren auf der Grundlage enzymgekoppelter-Antikörper bei bestimmten Phytophthora-Arten etabliert und auch kommerziell vertrieben (siehe MILLER, 1996; MILLER et al., 1997). Unerwünschte Kreuz-reaktionen mit anderen Mikroorganismen (beispielsweise mit der Gattung Pythium), eingeschränkte Sensitivität bei Wurzelproben sowie der enorme zeit- und gerätetechnische Aufwand behindern aber gegenwärtig den uneingeschränkten Einsatz dieser Systeme.

Alternativ wurden beispielsweise für Phytophthora parasitica, Phytophthora capsici, Phytophthora cinnamomi, Phytophthora megakarya und Phytophthora palmivora artspezifische Oligonukleotid-Hybridisierungssonden ent-wickelt (GOODWIN et al., 1989; LEE et al., 1993; JUDELSON und MESSENGER-ROUTH, 1996). Hier sind unerwartete Kreuzreaktionen oft ein Problem, weil die Artspezifität der publizierten Sonden oftmals nur gegenüber einer eng begrenzten Anzahl von Pathogenen getestet worden ist.

Durch den Einsatz der Polymerase-Kettenreaktion konnten schnelle, zuverlässige und einfache Testsysteme für zahlreiche Phytopathogene zur Verfügung gestellt werden (NAZAR et al., 1991; XUE et al., 1992; JOHANSON and JEGER, 1993; TISSERAT et al., 1994). Dabei handelt es sich um die in vitro-Synthese eines diagnostischen DNA-Markerfragmentes mit Hilfe von z.B. der thermostabilen bakteriellen Taq-DNA-Polymerase nach Bindung zweier artspezifischer Oligonuk-leotide an die genomische DNA des pathogenen Organismus. Auf dieser Grundlage hat man bereits artspezifische Primerpaare für den PCR-gestützten Nachweis von Phytophthora infestans, Phytophthora erythroseptica und Phytophthora nicotianae in infizierten Kartoffeln, Tabak sowie Tomatenpflanzen entwickelt (TOOLEY et al., 1997; LACOURT und DUNCAN, 1997).

Es war eine Aufgabe der vorliegenden Erfindung, einfache und sichere Nachweismethoden für Phytophthora citricola, Phytophthora cambivora und die erst kürzlich entdeckte Art Phytophthora quercina (JUNG, 1997) bereitzustellen, für deren experimentelle Durchführung kein mikrobiologisches Spezialwissen erforderlich ist. Die drei genannten Phytophthora-Arten wurden sehr häufig an verschiedenen geschädigten Eichen- sowie Buchenstandorten in Deutschland, Ungarn, Italien, Slowenien, Frankreich und der Schweiz isoliert.

Diese Aufgabe wird erfindungsgemäß durch die im Anspruch 1 näher gekennzeichneten PCR- sowie Hybridisierungsverfahren gelöst auf der Grundlage der erfindungsgemäß bereit-gestellten artspezifischen Nukleotidsequenzen für Phytophthora citricola, Phytophthora cambivora und Phytophthora quercina. Bevorzugte Ausgestaltungen der Erfindung ergeben sich aus den Unteransprüchen.

Erfindungsgemäß werden somit zum ersten Mal Oligonukleotid-Primer bereitgestellt, die einen spezies-spezifischen Nachweis und eine spezies-spezifische Quantifizierung von Phytophthora-Arten erlauben, die beispielsweise für Eichen und Buchen pathogen sind. Die erfindungsgemäß bereitgestellten Oligonukleotid-Primer sind hoch-spezifisch für den Nachweis von Phytophthora citricola, Phytophthora cambivora und Phytophthora quercina. Es ist jedoch möglich, einzelne oder mehrere Nukleotide der Oligonukleotid-Primer durch Addition, Deletion, Substitution und/oder chemische Modifikation zumindest eines Nukleotids zu verändern, wobei die Veränderung so durchgeführt werden muß, daß gleichwohl eine artspezifische Bindung an die genomische DNA von Phytophthora citricola, Phytophthora cambivora oder Phytophthora quercina beibehalten wird.

Von der Erfindung umfaßt werden auch solche Primer-Sequenzen, deren Basenfolge komplementär zu den erfindungsgemäß beanspruchten Oligonukleotid-Primersequenzen ist. Weiterhin werden von der Erfindung die von den Oligonukleotid-Primern abgeleiteten RNA-Sequenzen mitumfaßt. Die Erfindung umfaßt auch solche Oligonukleotid-Primer, die eine Sequenzhomologie von ≥ 90%, bevorzugt ≥ 95% zu den erfindungsgemäßen Primer-Sequenzen aufweisen. Weiterhin umfaßt werden solche Oligonukleotid-Primer, die unter stringenten Bedingungen mit der genomischen DNA. der oben genannten Phytophthora-Arten zur Hybridisierung gebracht werden können. Für alle genannten Abwandlungen gilt, daß nur solche von der Erfindung beansprucht werden, die eine spezies-spezifische Bindung an die genomische DNA von Phytophthora citricola, Phytophthora cambivora und Phytophthora quercina ermöglichen. Alle anderen Abwandlungen sind von der Erfindung nicht umfaßt.

Die bereitgestellten Oligonukleotid-Primer der Erfindung können entweder in einer Polymerase-Kettenreaktion (PCR) oder in einer Hybridisierungsreaktion zum artspezifischen Nachweis der genannten Phytophthora-Arten verwendet werden. Sowohl die PCR als auch die Hybridisierungsreaktion sind an sich bekannte Nachweisverfahren, die beispielsweise in Griffin und Griffin (1994) und Sambrook et al. (1989) beschrieben werden.

Das PCR-Verfahren hat den Vorteil, daß sehr kleine DNA-Mengen, beispielsweise in infizierten Wurzel- oder Bodenproben, nachweisbar sind. In Abhängigkeit von dem nachzuweisenden Material sind die Temperaturbedingungen und die Zykluszahlen der PCR abzuwandeln. Die optimalen Reaktionsbedingungen können durch Handversuche in an sich bekannter Weise ermittelt werden. Ein Beispiel für eine PCR ist wie folgt:
-
- Denaturierung der zu untersuchenden DNA-Probe bei 94°C mindestens 3 Minuten lang;
- Bindung der Primerpaare CITR1/CITR2, CAMB3/CAMB4 bzw. QUERC1/QUERC2 eine Minute lang bei 62°C an die beiden komplementären Einzelstränge der Untersuchungs-DNA;
- zwei Minuten Verlängerungsreaktion der einzelnen Primer entlang der DNA-Matrize bei 72°C durch Verknüpfung von Desoxyribonukleosidtriphosphaten mittels einer thermostabilen DNA-Polymerase;
- 30 bis 40 Reaktionszyklen jeweils bestehend aus: DNA-Denaturierung bei 94°C (30 Sekunden lang), gefolgt von Primerbindung bei 62°C (1 Minute lang), und Primerverlängerung bei 72°C (2 Minuten lang);
- Endreaktion zur Auffüllung der beiden 3'-Enden am Reaktionsprodukt bei 72°C (ca. 5-8 Minuten lang).

Die PCR-Reaktion kann in 25-100 Mikroliter Flüssigreaktionen oder an fixierten Gewebeschnittpräparaten im sogenannten *In situ*-Verfahren stattfinden.

Die im Verlauf der PCR-Amplifikation durch die Verlängerung der Primersequenzen entstandenen, charakteristischen, spezies-spezifischen DNA-Markerfragmente können beispielsweise gelelektrophoretisch oder fluorimetrisch unter Verwendung fluoreszenz-markierter Oligonukleotide nachgewiesen werden. Selbstverständlich sind auch andere, dem Fachmann bekannte Nachweisverfahren einsetzbar.

In einer alternativen Ausführungsform der Erfindung werden die artspezifischen Oligonukleotid-Primer der vorliegenden Erfindung jeweils einzeln mit der auf die Anwesenheit von Phytophthora citricola, Phytophthora cambivora oder Phytophthora quercina zu untersuchenden DNA-Probe zur Hybridisierung gebracht, wobei stringente Hybridisierungsbedingungen gewählt werden. Unter stringenten Reaktionsbedingungen wird erfindungsgemäß eine Hybridisierungstemperatur von ca. 5-10°C unter dem jeweiligen Primerschmelzpunkt verstanden. Die Stabilität der DNA/DNA- oder RNA/DNA-Hybriden muß selbst bei niedrigen Salzkonzentrationen entsprechend 0,1 x SSC/0,5% SDS gewährleistet sein. Auf diese Weise kann der Ablauf unerwünschter Kreuzreaktionen mit anderen Spezies verhindert werden. Die jeweiligen Temperaturbedingungen können jedoch in Abhängigkeit von den gewählten Versuchsbedingungen und in Abhängigkeit von der zu untersuchenden DNA-Probe unterschiedlich sein und müssen dann entsprechend angepaßt werden. Der Nachweis des Hybridisierungsprodukts kann beispielsweise durch Autoradiographie im Falle radioaktiv markierter Primermoleküle oder durch Fluormetrie bei Verwendung von fluoreszenz-markierten Oligonukleotiden erfolgen.

Die DNA der zu untersuchenden Phytophthora-Arten kann sowohl bei der PCR-Reaktion als auch bei der Hybridisierungsreaktion entweder in extrahierter Form vorliegen oder in Form von Schnittpräparaten biologischen Materials, so daß eine *In situ***-**Hybridisierung oder *In situ***-**PCR durchgeführt wird.

Nachfolgend wird die Erfindung anhand von Ausführungsbeispielen und anhand der beiliegenden Abbildungen näher beschrieben. Die Abbildungen zeigen:
- Abbildung 1:: Spezies-spezifische Amplifikation von P.citricola-DNA, P.cambivora-DNA und P.quercina-DNA;
- Abbildung 2:: eine PCR-gestützte Quantifizierung von P. cambivora-DNA, P.quercina-DNA und P.citricola-DNA;
- Abbildung 3:: den PCR-gestützten Nachweis von P.citricola-DNA in künstlich infizierten Buchenkeimlingen.

Die Tabelle 1 gibt eine Übersicht über die Bezeichnung, die Kulturnummer, die Quelle, die Wirtsorganismen sowie die Herkunft von verschiedenen Phytophthora- und Pythium-Spezies und anderen Phytopathogenen.

### Versuche zum Auffinden eines Phytophthora citricola-spezifischen DNA-Markers:

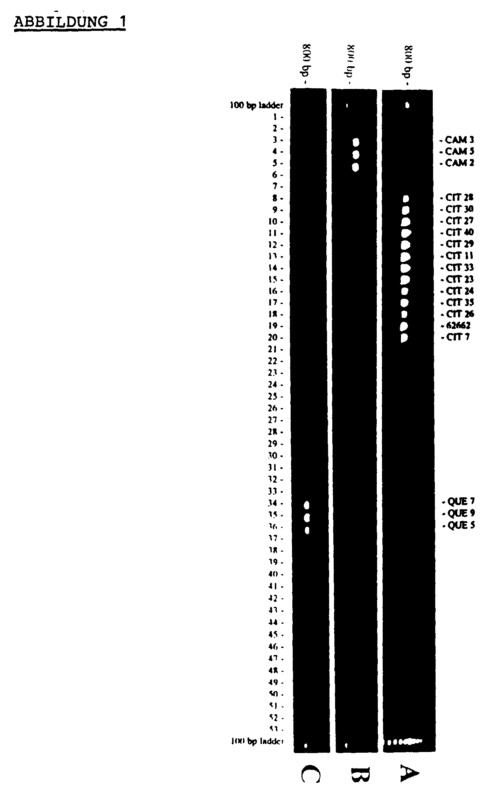

Die intern-transkribierten Spacer-Abschnitte ITS1 und ITS2 der ribosomalen RNA-Genrepeats von P. citricola (Isolat CIT 7), P. cambivora (Isolat CAM 2), P. gonapodyides (Isolat GON 3) und P. quercina (Isolat Que 7) wurden mit Hilfe der PCR auf der Basis des Oligonukleotidprimerpaares 5'-TCCGTAGGTGAACCTGCGG-3'/5'-TCCTCCGCTTATTGATATGC-3' (WHITE et al., 1990) amplifiziert, kloniert und anschließend in ihrer DNA-Sequenz aufgeklärt. Durch Vergleich der erhaltenen Daten wurden zwei für P. citricola-spezifische größere Deletionen im Bereich der ITS1- und ITS2-Region gefunden, die bereits durch COOKE and DUNCAN (1997) beschrieben worden sind. Auf der Grundlage dieser Sequenzunterschiede wurde das PCR-Primerpaar CITR1/CITR2 (5'-TCT TGC TTT TTT TGC GAG CC-3' und 5'-CGC ACC GAG GTG CAC ACA AA-3*'*) selektiert. Diese Primer-Kombination ergab für dreizehn getestete P. citricola-Isolate das erwartete PCR-Produkt von 711 Basenpaaren (siehe Abbildung 1). Von allen anderen überprüften Phytophthora-Spezies und weiteren Phytopathogenen, die in der Tabelle 1 aufgelistet sind, wurden keinerlei sichtbare PCR-Produkte erhalten. Besonders bemerkenswert ist, daß unter den beschriebenen Bedingungen auch keine Kreuzreaktionen mit P. citrophthora, P. capsici und P. cryptogea auftraten, die im Bereich der ITS-Regionen mit P. citricola eng verwandt sind (COOKE and DUNCAN, 1997).

### Entwicklung spezies-spezifischer DNA-Marker für Phytophthora cambivora und Phytophthora quercina:

Die ursprünglich gestarteten Versuche, für P. cambivora und P. quercina artspezifische Primerpaare auf der Grundlage der ITS1- und ITS2-Region zu entwickeln, scheiterten, weil die DNA-Sequenzenhomologien vieler Phytophthora-Arten in diesem Genombereich sehr ausgeprägt sind (siehe COOKE and DUNCAN, 1997). Die auf der Basis von ITS-Sequenzen selektierten PCR-Primerkombinationen zeigten Kreuzreaktionen mit anderen Phytophthora-Spezies, aber auch mit Pythium-Arten und sogar mit Armillaria-Organismen.

Mit Hilfe der PCR konnten unter Verwendung von kurzen DNA-Primern zufälliger Nukleotidsequenz (geliefert von der Firma OPERON Technologies/USA) jeweils ein für P. citricola-spezifisches sowie ein für P. cambivora-spezifisches subgenomisches DNA-Fragment amplifiziert werden. Beide Fragmente wurden kloniert und in ihrer DNA-Folge aufgeklärt, wobei sich keinerlei Übereinstimmung mit solchen DNA-Sequenzen zeigte, die bereits in inter-nationalen elektronischen Datenbanken vorhanden sind. Ausgehend von den terminalen DNA-Sequenzen beider Fragmente wurde jeweils das PCR-Primerpaar CAMB3/CAMB4 (5*'*-GTG ACG TAG GTT CAT CTG CT-3*'* und 5*'*-GTG ACG TAG GCC AAA ATA ACA-3*'*) sowie das PCR-Primerpaar QUERC1/QUERC2 (5*'*-GTG ATC GCA GGA GTG CTC TT-3*'* und 5*'*-GTG ATC GCA GTA AGA AAT GAG T-3*'*) ausgewählt. Die Primer-Kombination CAMB3/CAMB4 lieferte ein 1105 Basenpaare umfassendes PCR-Produkt mit P. cambivora-DNA, wohingegen mit dem Primerpaar QUERC1/QUERC2 ein 842 Basenpaare langes PCR-Produkt unter Verwendung von P. quercina-DNA erhalten worden ist (siehe Abbildung 1). Beide Primerpaare zeigten keinerlei Kreuzreaktionen mit den in der Tabelle 1 aufgeführten und getesteten Spezies.

### Quantifizierung und Sensitivität des Nachweises:

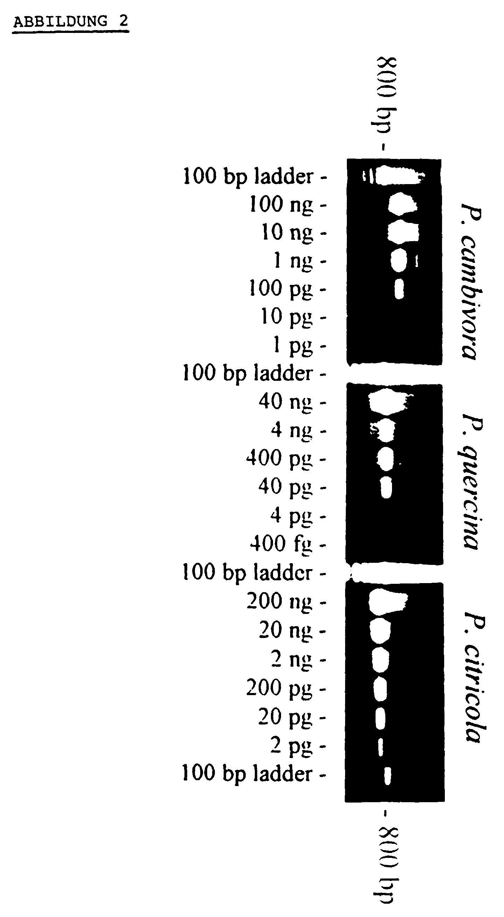

Isolierte genomische DNA-Proben von P. citricola (200 ng/µl Ausgangskonzentration), P. cambivora (100 ng/µl Ausgangs-konzentration) und P. quercina (40 ng/µl Ausgangskonzentration) wurden schrittweise mit TE-Puffer (10 mM Tris/1mM EDTA; ph 8,0) jeweils um den Faktor 10 verdünnt. Sichtbare Amplifikate sind mit diesen Proben unter den getesteten PCR-Reaktionsbedingungen (beschrieben in Material und Methoden; 30 PCR-Zyklen) bei P. citricola bis zu einer DNA-Konzentration von 2.0 pg, bei P. quercina bis zu einer DNA-Konzentration von 4.0 pg und im Fall von P. cambivora bis zu einer Konzentration von 100 pg erhalten worden (siehe Abbildung 2). Dies heißt, daß die untere PCR-Nachweisgrenze dem DNA-Äquivalent von ca. 2 P. citricola-Kernen, 4 p. quercina-Kernen und ca. 100-200 P. cambivora-Kernen entspricht. Dabei wird unterstellt, daß bei allen drei Spezies genauso wie im Fall von P. infestans 10 pg genomische DNA etwa 10-20 Kernäquivalenten entsprechen (TOOLEY and THERRIEN, 1987).
Die ermittelten, teilweise erheblichen Differenzen in der Testempfindlichkeit werden möglicherweise durch eine unterschiedliche Kopienzahl jener Genomabschnitte verursacht, an welche die entwickelten artspezifischen PCR-Primerpaare binden. Wahrscheinlich besitzen jene Genombschnitte, die von den Primerpaaren CAMB3/CAMB4 erkannt werden, nicht die hohe Redundanz mit der die subgenomischen Erkennungsfragmente der Primerpaare CITR1/CITR2 und QUERC1/QUERC2 im Zellkern der betreffenden Phytophthora-Spezies vorliegen.

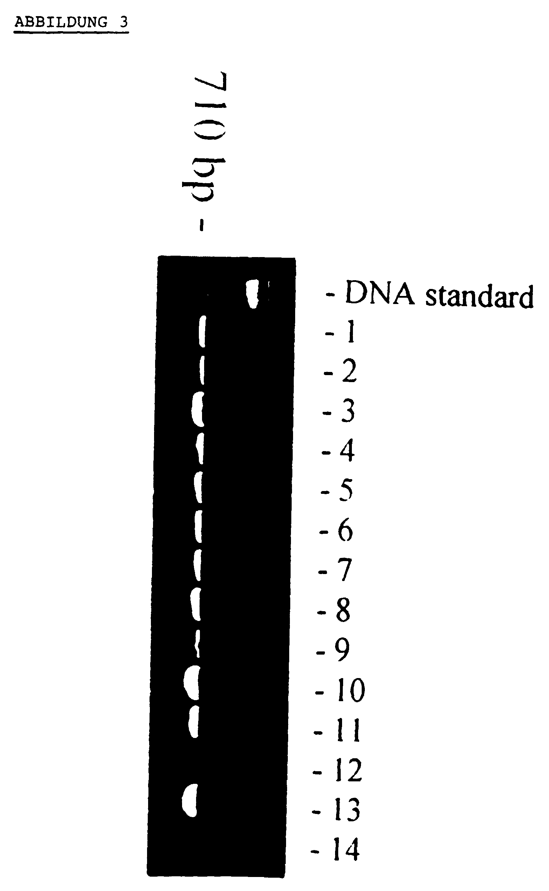

Die PCR-Sensitivität war nach Zugabe von 100 ng isolierter Eichenwurzel-DNA um den Faktor 10 geringer, wie am Beispiel von P. citricola-DNA exemplarisch gefunden worden ist (Daten nicht gezeigt). Offenbar liegen noch PCR-inhibitorische Substanzen in den Wurzelextrakten vor, was sich vielleicht durch verbesserte DNA-Isolationsprotokolle verhindern läßt. Alternativ kann man die Nachweisgrenze für die einzelnen Phytophthora-Organismen durch eine Erhöhung der PCR-Zyklusanzahl steigern. Die erreichte Testempfindlichkeit war aber völlig ausreichend, um P. citricola, P. cambivora bzw. P. quercina in künstlich infizierten Eichen- bzw. Buchenkeimlingen am dritten Tag nach der Inokulation sicher nachzuweisen (exemplarisch für P. citricola in Abbildung 3 gezeigt). Die Phytophthora-Infektionen konnten auf diese Weise sowohl in symptomatischen als auch in nicht-symptomatischen Gewebeproben der infizierten Keimlinge bestätigt werden.

Erfindungsgemäß werden somit Nukleotidsequenzen zur Verfügung gestellt, die als Primerpaare im PCR-Verfahren einsetzbar sind und somit einen einfachen, schnellen und sicheren artspezifischen Nachweis von P. citricola, P. quercina und P. cambivora erlauben, eine Quantifizierung des Pathogens sowie die Unterscheidung von anderen Phytophthora-Arten ermöglichen. Grundlage bildet das in seinen Einzelheiten bekannte automatisierte PCR-Verfahren (ausführlich beschrieben von GRIFFIN und GRIFFIN,1994), daß durch den wiederholten Zyklus von DNA-Denaturierung, Primer-Bindung und DNA-Neusynthese zu einer sichtbaren exponentiellen Amplifikation von DNA-Markerfragmenten führt. Beim erfindungsgemäßen Verfahren wird beispielsweise die genomische DNA einer biologischen Untersuchungsprobe isoliert und anschließend durch Erwärmung, bevorzugt bei 94°C, in beide Einzelstränge denaturiert. Nach Abkühlung auf 62°C läßt man zwei einzelsträngige Oligonukleotid-primer an die Matrizen-DNA binden. Die Kettenverlängerung der Primer komplementär den einzelsträngigen DNA-Matrizen findet nach Erwärmung bei 72°C in Anwesenheit einer hitzestabilen DNA-Polymerase statt. Der Reaktionszyklus wird 30-40 mal durchlaufen. Die synthetisierten diagnostischen DNA-Markerfragmente lassen sich dann mit geeigneten Verfahren (z. B. Agarose-Gelelektrophorese) nachweisen. Dieses PCR-Verfahren wurde für die Detektion von baumpathogenen Phytophthora-Arten adaptiert, indem Nukleotidsequenzen ermittelt worden sind, die spezifisch an Genomabschnitte von P. citricola, P. cambivora und P. quercina binden und keinerlei störende Kreuzreaktionen mit den Genomen anderer Phytopathogene zeigen. Die DNA muß bei dem PCR-Verfahren nicht unbedingt extrahiert werden, sondern kann auch in Gewebeschnitten fixiert sein, was einen direkten Nachweis in den Wirtszellen ermöglicht.

Es wird insbesondere darauf hingewiesen, daß die Erfindung nicht auf die vorstehend beschriebenen und beanspruchten Nukleotidsequenzen beschränkt ist. Die obigen Sequenzen können durch Addition, Deletion und chemische Modifikation von Nukleotiden abgeändert werden. Außerdem können die komplementären DNA-Sequenzen oder abgeleitete RNA-Sequenzen eingesetzt werden, wobei jedoch nur solche Veränderungen umfaßt werden, die den erfindungsgemäßen, artspezifischen Nachweis von P. quercina, P. cambivora und P. citricola nicht beeinträchtigen. Außerdem lassen sich die beanspruchten DNA-Sonden auch einzeln im sog. Hybridisierungsverfahren anwenden. Hierbei bilden die einzelsträngigen Oligonukleotidprimer mit denaturierter Phytophthora-DNA artspezifische Hybride, die beispielsweise autoradiographisch nachweisbar sind. Hierbei muß die Anwendung stringenter Reaktionsbedingungen (Hybridisieruns-temperatur vorzugsweise nicht niedriger als 5-10°C unterhalb der Primerschmelzpunkte) den Ablauf von unerwünschten Kreuzreaktionen sicher ausschließen, wobei das doppelsträngige DNA/DNA- bzw. RNA/DNA- Hybridmolekül auch unter geringen Salzkonzentrationen (vorzugsweise 0.1xSSC/0.5%SDS) noch stabil sein sollte. Die optimalen, individuellen Hybridisierungstemperaturen unter denen ausschließlich artspezifische Nachweisreaktionen statt-finden, können ohne erfinderisches Zutun empirisch ausgetestet werden.

Mit den beanspruchten Verfahren können Infektionen von P. citricola, P. quercina und P. cambivora bereits vor dem Erscheinen sichtbarer Schäden in symptomlosen Geweben erfaßt werden. Diese Erfassung ist nicht nur wichtig für ein Massenscreening natürlicher Waldstandorte. Da Buchen- sowie Eichenbestände im großen Ausmaß angepflanzt werden, muß in Zukunft unbedingt eine Verbreitung der verschiedenen Phytophthora-Pathogene auf der Grundlage von Baumschul-material verhindert werden. Außerdem bietet sich eine Suche nach eventuell vorhandenen Phytophthora-resistenten Baum-genotypen an. Mit Hilfe der erfindungsgemäß bereitgestellten Quantifizierungsmethoden läßt sich u.a. prüfen, ob nach einer künstlichen Infektion vielleicht bestimmte Baumgenotypen die Vermehrung von Phytophthora-Pathogenen abwehren. Weiterhin kann die Wirksamkeit phytosanitärer Schutzmaßnahmen und die Wirksamkeit der gentechnischen Übertragung von Abwehrgenen kontrolliert werden.

### MATERIAL und METHODEN

### Pilzisolate, Kulturbedingungen und DNA-Extraktionsverfahren

Die Isolate von Phytphthora, Pythium, Xerocomus, Hebeloma, Russula, Armillaria und Heterobasidion, die bei den vorliegenden Untersuchungen verwendet wurden, die Isolat-Nummern, Quellen-angaben und die Wirtsorganismen sind in der Tabelle 1 angegeben.

Die Phytophthora-Arten und andere Pilze wurden bei 15°C in 1 ml sterilem, destillierten Wasser auf Stücken aus 2% Malzextrakt-Agar gezogen, die aus einer überwachsenen Petrischalen-Kultur mit einer sterilen Pasteurpipette ausgestanzt wurden. Das Mycel zur DNA-Extraktion wurde in 15 ml flüssigem 2% Malzextraktnährmedium bei 20°C gezogen. Die zur Produktion infektiöser Zoosporen verwendeten Kulturen wurden auf V8-Flüssigagar gezogen.

Die DNA aus den Mycelien als auch den Baumwurzeln kann, wie nachfolgend beschrieben, extrahiert werden.

### Künstliche Samenkultivierung und DNA-Extraktion aus infiziertem Pflanzenmaterial

Samen von Eiche (Q. robur) und Buche (F. sylvatica) wurden zwei Wochen lang in Kunststoffbehältern bei Raumtemperatur auf feuc-htem, sterilen Vermiculit oder feuchtem Filterpapier gehalten. Nach dem Waschen mit demineralisiertem Wasser wurden Keimlinge mit 1 bis 3 cm langen Keimwurzeln in mit Parafilm verschlossene Teströhrchen überführt, die mit sterilem Leitungswasser (20 ml) vollständig gefüllt waren. Nach 2-wöchiger Inkubation bei Raumtemperatur und Dunkelheit wurden die Keimlinge mit den Pilzen infiziert, wenn die primären Wurzeln durch die Perforation der Parafilm-Membran gewachsen waren und eine Länge von 15 bis 20 cm (Eiche) oder 10 bis 15 cm (Buche) erreicht hatten.

Infektionsuntersuchungen wurden mit den P. citricola-Isolaten CIT 23/CIT 55, den P. quercina-Isolaten QUE 3/QUE 4 und dem P. cambivora-Isolat CAM 5 mit den nachfolgenden zwei verschiedenen Methoden durchgeführt. Bei der ersten Methode wurden die Keimlinge aufrecht in 100 ml Glasteströhrchen (Eiche) oder 50 ml Glasfläschchen (Buche) eingebracht, wobei die Röhrchen bzw. Fläschchen steriles, demineralisiertes Wasser und 5 Agarstückchen enthielten, die aus den Wachstumsenden einer eine Woche alten Phytophthora-Kultur erhalten wurden. Zu diesem Zweck wurden die Glasgefäße mit Parafilm verschlossen, und die Wurzeln der Sämlinge wurden durch ein Loch im Parafilm eingeführt. Die Wurzelspitzen kamen nie in direkten Kontakt mit den Agarstückchen.

Bei der zweiten Methode wurden die Sämlinge in 1,1 l Kunststoffgefäße (10 x 20 x 8 cm) mit 250 ml sterilem, demineralisierten Wasser und fünf Agarstückchen aus den oben beschriebenen Phytophthora-Kulturen überführt. Die Keimlinge und die jungen Sprößlinge wurden über dem Wasserniveau fixiert. Weiterhin wurden die Wurzeln so befestigt, daß kein direkter Kontakt mit den Agarstückchen erfolgte, die Wurzeln aber weiterhin unterhalb der Wasseroberfläche verblieben. Die Behälter wurden verschlossen, um eine übermäßige Verdunstung zu vermeiden. Alle Testgefäße wurden unter Tageslicht bei 18°C gehalten. Nach drei Tagen begannen sich sichtbare Krankheitssymptome zu entwickeln, und die für die Infektion benutzten Phytophthora-Arten wurden reisoliert, um die Infektion zu bestätigen. Hierfür wurden kleine Stücke der Gewebe täglich geerntet, mit demineralisiertem Wasser gewaschen, auf Filterpapier getrocknet und auf Agar-Selektivmedium mit Hymexazol (PARPNH) (TSAO, 1982) plattiert. Die Agarplatten wurden bei 20°C im Dunklen inkubiert und täglich mikroskopisch untersucht. Die sich entwickelnden Phytophthora-Kolonien wurden auf V-8 Agar übertragen und kultiviert.

DNA-Extraktionen von erkrankten Wurzelspitzen, Proben ohne Symptome und nichtinfizierten Kontrollen wurden getrennt für jeden Probenzeitpunkt nach Infektion unter Verwendung von DNeasy Plant Mini Kit (QIAGEN) gemäß den Angaben des Herstellers durchgeführt. Für die Buchen wurden die unterschiedlichen Testgewebe (Wurzeln, Hypocotyle und Cotyledonen) getrennt extrahiert. Proben mit einem Naßgewicht von 15 bis 100 mg wurden in flüssigem Stickstoff unter Verwendung eines Mörsers zerkleinert und umgehend in Eppendorf -Röhrchen überführt, die 400 ml Lysispuffer und 2,5 mg Polyvinylpyrrolidon enthielten. Alternativ wurden die Proben direkt in den Eppendorf-Röhrchen unter Verwendung von flüssigem Stickstoff und eines Glasstabes zerkleinert. Der Lysispuffer (QIAGEN) wurde mit 50mM Ascorbinsäure ergänzt.

Nach Bindung an eine Silicagel-Säule (QIAGEN) wurde die DNA gemäß den Angaben des Herstellers eluiert und bei 4°C bis zur PCR-Amplifikation gelagert. Das beschriebene QIAGEN-Verfahren wurde auch verwendet, um DNA aus dem Phytophthora-Mycel zu extrahieren, um für die PCR-Diagnostik eine positive Kontrolle zu haben.

### PCR-Primer, DNA-Amplifikation und Produktanalyse

In der Tabelle 2 sind die Nukleotidsequenzen für alle bei den vorliegenden Untersuchungen verwendeten Primer aufgeführt. Die ribosomalen ITS-Fragmente wurden unter Verwendung des von WHITE et al. (1990) beschriebenen Primer-Paares ITS1/ITS2 amplifiziert. Zufallsverteilt-amplifizierte polymorphe DNA (RAPD)-PCR wurde in 25 ml Reaktionsansätzen erhalten, die 50 mM KCl, 2,0 mM MgCl₂, 10 mM Tris/HCl, pH 9,0, 100 mM je dNTP (PHARMACIA), 0,2 mM eines RAPD-Primers (OPERON Technologies Inc., Alameda, USA: Primer OPA 1 - OPA 20), 0,4 mg BSA (DNase-frei, PHARMACIA), 1 Einheit Taq DNA-Polymerase (PHARMACIA) und ungefähr 25 ng Template-DNA enthielten. Die Reaktionsbestandteile wurden in Form von Stammischungen hergestellt, um Fehler beim Pipettieren und das Risiko einer DNA-Kontamination zu minimieren. In 96-Well-Thermoschalen wurden 24 ml Aliquots der Ansatzmischungen pipettiert, wozu 1 ml Template-DNA zugegeben wurden, und das ganze wurde mit 25 ml Siliconöl (Typ3, MERCK, Deutschland) überschichtet, um eine Verdunstung zu verhindern. Die Thermocycler (Uno, BIOMETRA, Deutschland)-Bedingungen waren wie folgt:

Eingangs-Denaturierung bei 94°C ( 3 Min.), Annealing der Primer bei 40°C (1 Min.) und Primer-Extension bei 72°C (2 Min.), nach-folgend 35 Zyklen bei 94°C (30 Sek.), 40°C ( 45 Sek.) und 72°C (2 Min.). Eine abschließende Elongation wurde 5 Min. bei 72°C durchgeführt, um ein doppelsträngiges Amplicon zu erhalten. Nach Zugabe von 5 ml Gel-Beladungspuffer wurden PCR-Produkte durch horizontale Agarose-Gelelektrophorese analysiert. Der oben beschriebene 25 ml Reaktionsansatz enthielt 0,1 bis 200 ng Template-DNA und 0,2 mM eines jeden Primers und wurde zur Amplifikation Phytophthora-spezifischer Markerfragmente zu diagnostischen Zwecken unter den nachfolgenden PCR-Bedingungen verwendet:
Anfangs-Denaturierung bei 94°C (3 Min.), Annealing der Primer bei 62°C (1 Min.) und Primer-Extension bei 72°C (2 Min.), nachfolgend 30 Zyklen bei 34°C (30 Sek.), 62°C (1 Min.) und 72°C (2 Min.). Ein abschließender Verlängerungsschritt wurde bei 72°C 5 Min. lang durchgeführt. Nach Zugabe von 5 ml des Gel-Beladungs-puffers wurden die Amplicone durch horizontale Agarose-Gelelektrophorese, wie bei SCHULZE et al. (1997) beschrieben, analysiert.

### Klonierung von PCR-Produkten und DNA-Sequenzierung

Aus mit Ethidiumbromid gefärbtem Agarose-Gel wurden die spezifischen PCR-Banden unter Verwendung des QIAquick-Gelextraktionskits (QIAGEN) gemäß den vom Hersteller angegebenen Bedingungen extrahiert. 2 ml der aus dem Gel isolierten DNA (300 ng) wurden mit 1 ml Novagen pT7 Blue T-Vektor (50 ng), 1 ml 10x Ligationspuffer (NOVAGEN), 0,5 ml 100 mM DTT, 0,5 ml 10 mM ATP, 4,5 ml nukleasefreiem Wasser, 0,5 ml (1 Einheit) T4 DNA Ligase (NOVAGEN) vermischt und bei 16°C 12 Stunden lang inkubiert. Kompetente NovaBlue E.coli K12-Zellen (NOVAGEN) wurden mit 1 ml Aliquot der Ligationsreaktion gemäß den Bedingungen des Herstellers transformiert. Rekombinante Bakterienzellen wurden nach Übernachtinkubation bei 37°C auf LB Agarplatten mit 50 mg/ml Ampicillin, 15 mg/ml Tetracyclin, 35 ml von 50 mg/ml X-Gal (aufgelöst in Dimethylformamid) und 20 ml 100 mM IPTG selektiert.

Die DNA der zufällig gepickten rekombinanten pT7 Blue T-Plasmidderivate wurde aus flüssigen Bakterienkulturen unter Verwendung des QIAGEN-Plasmidminikits und von QIAGEN-tip 20-Säulen präpariert. Beide DNA-Stränge der Plasmide wurden mit Hilfe einer Oligonukleotid-Walking-Strategie unter Verwendung des Cy5-AutoRead-Sequenzierungskits und des Cy5-dATP Markierungsgemisches (PHARMACIA) sequenziert. Durch Didesoxynukleotide terminierte Fluoreszenz-markierte Fragmente wurden durch eine automatisierte Lasersequenzierungsvorrichtung ALF express (PHARMACIA) nachgewiesen. Klonspezifische Oligonukleotide wurden von GIBCO/BRL synthetisiert.

Die erfindungsgemäß bereitgestellten Primer-Sequenzen können in der oben angegebenen Weise modifiziert werden. Insbesondere werden von der Erfindung mitumfaßt Primer-Sequenzen, die mit den Sequenzen nach Anspruch 1 eine Homologie von mindestens 95 %, beispielsweise 96, 97, 98 und 99 %, aufweisen. Der Fachmann kann die erfindungsgemäß offenbarten Primersequenzen durch Austausch eines Nukleotids oder durch Austausch mehrerer Nukleotide abändern und durch nachfolgende Hybersidierungsexperimente feststellen, ob weiterhin eine artspezifische Bindung an die genannten Phytophthora-Arten möglich ist.

Beim Abändern der Primer-Sequenzen wird der Fachmann berücksichtigen, daS für die erfolgreiche Amplifizierung eines Primers das paßgenaue Annealing des 3'-Endes des Primers an eine Target-DNA (Matrize, genomische DNA) notwendig ist. Am 3'-Ende ist somit für ca. 3 - 5 Basen eine exakt passende Sequenz erforderlich. Zum 5'-Ende nimmt die Stringenz diese Bedingungen jedoch ab. So können von den ersten 5 Basen des 5'-Endes 1 oder 2 Basen ausgetauscht werden, ohne die Spezifität zu verändern bzw. mit nur minimalen Veränderungen, während von den Basen 5 - 15 ab dem 5'-Ende ohne Spezifitätsverlust in der Regel nur 1 Base austauschbar ist. Unter Berücksichtigung dieser Überlegungen kann der Fachmann die im Anspruch 1 genannten Primer abändern,um weitere erfindungsgemäß einsetzbare Primer zu erhalten.

### Literaturverzeichnis:

BLASCHKE H (1994) Decline symptoms on roots of *Quercus robur*. Eur. J. For. Path. **24**: 386-398.
BRASIER CM (1993) *Phytophthora cinnamomi* as a contributory factor in European oak declines. In: LUISI N, LERARIO P and VANNINI A (eds.) Recent Advances in Studies on Oak Decline. Proc. Int. Cong., Selva di Fassano (Brindisi), Italy, Sep. 13-18, 1992, Tipolitografia-Putignano. pp.49-57.
COOKE DEL and DUNCAN JM (1997) Phylogenetic analysis of *Phytophthora* species based on ITS1 and ITS2 sequences of the ribosomal RNA gene repeat. Mycol. Res**. 101**: 667-677.
ERWIN DC and RIBEIRO OK (eds.) (1996) *Phytophthora* diseases world-wide. (562 pp.) St. Paul, Minnesota, APS Press**.**
GOODWIN PH, KIRKPATRICK BC and DUNIWAY JM (1989) Cloned DNA probes for identification of *Phytophthora parasitica*. Phytopathology **79**: 716-721.
GRIFFIN HG and GRIFFIN AM (1994) PCR technology Current Innovations.CRC Press, Boca Raton, Ann Arbor, London, Tokyo, 370 p.
JOHANSON A and JEGER MJ (1993) Use of PCR for detection of *Mycosphaerella fijiensis* and *M. musicola,* the causal agents of Sigatoka leaf spots on banana and plantain. Mycol. Research **97**: 670-674.
JUDELSON HS and MESSENGER-ROUTH B (1996) Quantitation of *Phytophthora cinnamomi* in avocado roots using a species-specific DNA probe. Phytopathology **86**: 763-768.
JUNG T, BLASCHKE H and NEUMANN P (1996) Isolation, identification and pathogenicity of *Phytophthora* species from declining oak stands. Eur. J. For. Path**. 26**: 253-272.
LACOURT I and DUNCAN JM (1997) Specific detection of *Phytophthora nicotianae* using the polymerase chain reaction and primers based on the DNA sequence of its elicitin gene ParA1. Eur. J**.** Plant Path. **103:** 73-83.
LEE SB, WHITE TJ and TAYLOR JW (1993) Detection of *Phytophthora* species by oligonucleotide hybridization to amplified ribosomal DNA spacers. Phytopathology **83**: 177-181.
MILLER SA (1996) Detecting propagules of plant pathogenic fungi. Advances in Botanical Research **23:** 73-102.
MILLER SA, MADDEN LV and SCHMITTHENNER AF (1997) Distribution of *Phytophthora* spp*.* in field soils determined by immuno-assay. Phytopathology **87:** 101-107.
NAZAR RN, HU X, SSCHMIDT J, CULHAM D and ROBB J (1991) Potential use of PCR-amplified ribosomal intergenic sequences in the detection and differentiation of *Verticillium* wilt pathogens. Phys. and Mol. Plant Pathology **39:** 1-11.
NEWHOOK FJ, WATERHOUSE GM and STAMPS DJ (1978) Tabular key to the species of *Phytophthora* DE BARY. Mycological Paper 143, Commonwealth Mycological Institute: Kew**.** 20 pp.
Sambrook, J, E.F. Fritsch and T. Maniatis (1989), Molecular Cloning. A laboratory manual. Cold Spring Harbor Laboratory Press.
SIWECKI R and LIESE W (1991) Oak decline an Europe**.** In: SIWECKI R and LIESE W (eds.) Proc. Int. Symp., Kornik, Poland, May 15-18, 1990. pp.360.
STAMPS DJ, WATERHOUSE GM, NEWHOOK FJ and HALL GS (1990) Revised tabular key to the species of *Phytophthora.* 2nd ed., Mycological Papers 162, CAB International Mycological Institute: Wallingford, Oxfordshire, England**.**
TISSERAT NA, HULBERT SH and SAUER KM (1994) Selective amplification of rDNA internal transcribed spacer regions to detect *Ophiosphaerella korrae* and *O*. *herpotricha.* Phytopathology **84:** 478-482.
TOOLEY, PW and THERRIEN, CD (1987) Cytophotometric determination of the nuclear DNA content of 23 Mexican and 18 non-Mexican isolates of *Phytophthora infestans*. Exp. Mycol. **11**: 19-26.
TOOLEY PW, BUNYARD BA, CARRAS MM and HATZILOUKAS E (1997) Development of PCR primers from internal transcribed spacer region 2 for detection of *Phytophthora* species infecting potatoes. Appl. and Environm. Microbiology **63**: 1467-1475.
TSAO PH (1983) Factors affecting isolation and quantitation of *Phytophthora* from soil. In: ERWIN DC, BARTNICKY-GARCIA S and TSAO PH (eds.) *Phytophthora.* Its Biology, Taxonomy, Ecology and Pathology (pp. 219-236) American Phytopathological Society, St Paul, Minnesota.
WHITE TJ, BRUNS T, LEE, S and TAYLOR J (1990) Amplification and direct sequencing of fungal ribosomal RNA genes for phylogenetics. In: INNIS MA, GELFAND DH, SNINSKY JJ and WHITE TJ (eds.) PCR protocols: A guide to methods and applications (pp. 315-321) Academic Press Inc. New York.
XUE B, GOODWIN PH and ANNIS SL (1992) Pathotype identification of *Leptosphaeria maculans* with PCR and oligonucleotide primers from ribosomal internal transcribed spacer sequences. Phys. and Mol. Plant Pathology **41:** 179-188**.**

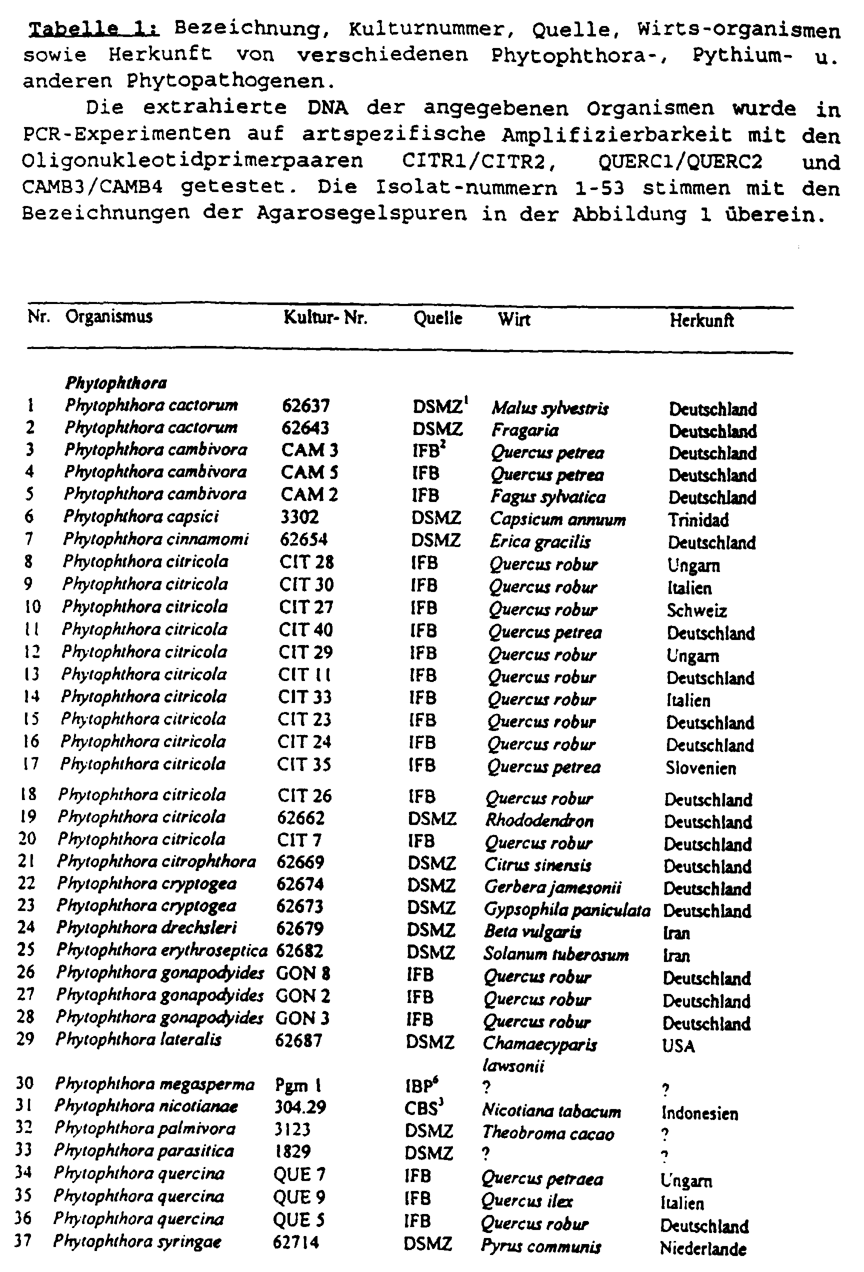

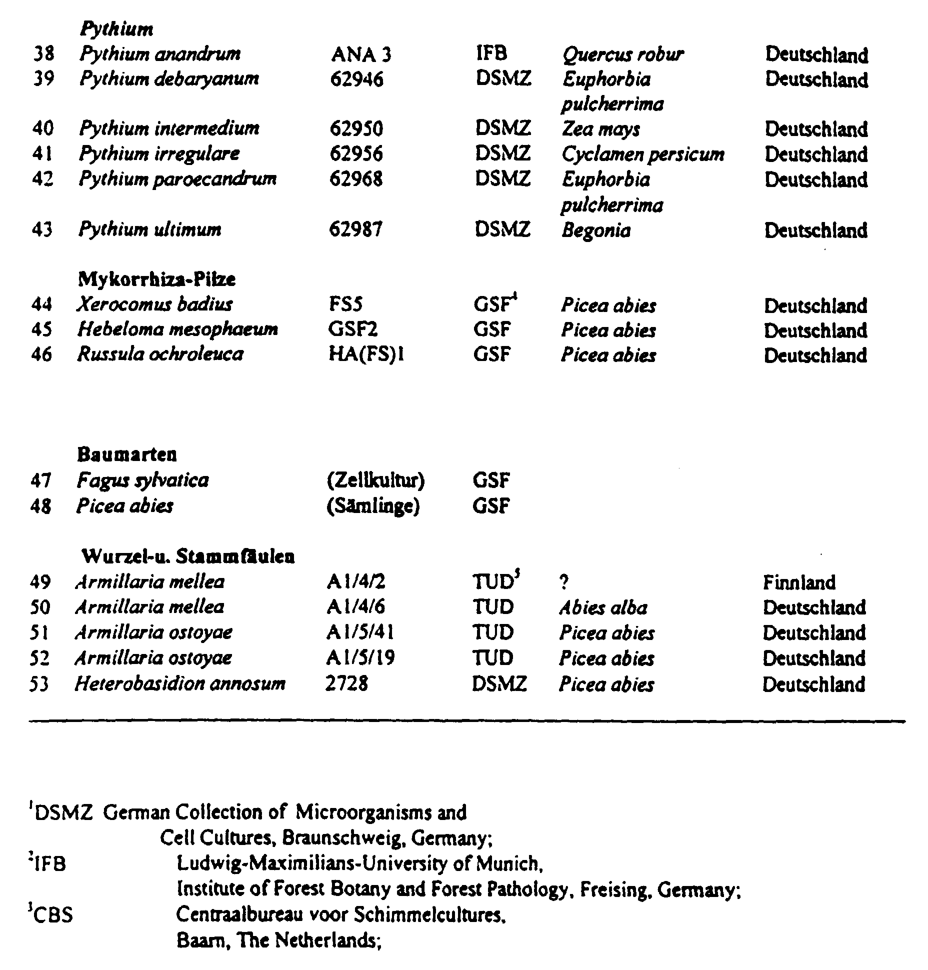

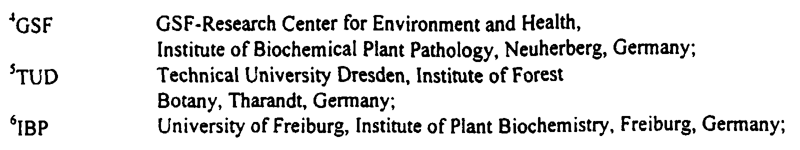

**TABELLE 2**

| Primer | Nukleotidsequenz | Quelle |
|---|---|---|
| ITS1 | 5'TCCGTAGGTGAACCTGCGG3' | White et al., 1990 |
| ITS4 | 5'TCCTCCGCTTATTGATATGC3' | White et al., 1990 |
| OPA8 | 5'GTGACGTAGG3' | OPERON Technologies |
| OPA10 | 5'GTGATCGCAG3' | OPERON Technologies |
| CITR1 | 5'TCTTGCTTTTTTTGCGAGCC3' | |
| CITR2 | 5'CGCACCGAGGTGCACACAAA3' | |
| CAMB3 | 5'GTGACGTAGGTTCATCTGCT3' | |
| CAMB4 | 5'GTGACGTAGGCCAAAATAACA3' | |
| QUERC1 | 5'GTGATCGCAGGAGTGCTCTT3' | |
| QUERC2 | 5'GTGATCGCAGTAAGAAATGAGT3' | |

**Abbildung 1:** Spezies-spezifische Amplifikation von P. citicola-DNA (Teil A; Spur 8-20) mit Hilfe des PCR-Primerpaares CITR1/CITR2, von P. cambivora-DNA (Teil B; Spur 3-5) mit Hilfe des PCR-Primerpaares CAMB3/CAMB4 bzw. von P. quercina-DNA (Teil C; Spur 34-36) unter Verwendung des PCR-Primerpaares QUERC1/QUERC2. Die DNA-Markerfragmente wurden nach erfolgter PCR im 1% Ethidiumbromid-gefärbten Agarosegel elektrophoretisch aufgetrennt und anschließend unter UV-Licht sichtbar gemacht. Alle drei entwickelten Oligonukleotid-primerpaare zeigen keinerlei unerwünschte Kreuz-reaktionen mit den genomischen DNA-Proben von jenen anderen Organismen, die in der Tabelle 1 aufgeführt worden sind. Die Bezeichnung aller in den Agarosegelspuren 1-53 untersuchten Organismen ist mit den angegebenen Isolatnummern in der Tabelle 1 identisch.
**Abbildung 2:** PCR-gestützte Quantifizierung von P. cambi-vora-DNA mit Hilfe des Primerpaares CAMB3/CAMB4, von P. quercina-DNA mit Hilfe des Primerpaares QUERC1/QUERC2 bzw. von P. citricola-DNA, unter Verwendung des Oligo-nukleotidprimerpaares CITR1/CITR2. Die DNA-Marker-fragmente wurden nach erfolgter PCR im 1% Ethidium-bromid-gefärbten Agarosegel elektrophoretisch aufge-trennt und anschließend unter UV-Licht sichtbar gemacht. Die Signalstärke des nach 30 PCR-Zyklen erhaltenen Amlicons ist von der eingesetzten Menge an Phytophthora-DNA sowie von dem genomischen Kopiengrad jener Sequenzen abhängig, welche von den entwickelten artspezifischen Primerpaaren erkannt werden. Die Zahlenangaben unterhalb der Gelspuren (400 fg-200 ng) beziehen sich auf die DNA-Menge, die in der PCR vorlag.
**Abbildung 3:** PCR-gestützter Nachweis von P. citricola-DNA in künstlich infizierten Buchenkeimlingen mit Hilfe des species-spezifischen Primerpaares CITR1/CITR2. Die diagnostischen DNA-Markerfragmente wurden nach 40 PCR-Zyklen im 1% Ethidiumbromidgefärbten Agarosegel elek-trophoretisch aufgetrennt und anschließend unter UV-Licht sichtbar gemacht. Die Durchführung der Pathogeninfektion am Tag 0 erfolgte wie im Abschnitt 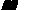 MATERIAL und METHODEN" beschrieben (Methode 1: Gelspuren 7-11 bzw. Methode 2: Gelspuren 1-6). Nach der Inokulation wurde DNA aus folgenden Gewebeproben extrahiert und in der PCR eingesetzt: Spur 1-symptomatische Hauptwurzel am Tag 3; Spur 2- symptomatisches Hypokotyl am Tag 3; Spur 3- symptomatisches Keimblatt am Tag 3; Spur 4- symptomatische Hauptwurzel am Tag 4; Spur 5- Hauptwurzel mit schwachen Krannheitssymptomen am Tag 4; Spur 6- symptomatische Hauptwurzel am Tag 6; Spur 7- sympto-matische Hauptwurzel am Tag 4; Spur 8- symptomlose Haupt-wurzel am Tag 4; Spur 9- symptomatische Hauptwurzel am Tag 5; Spur 10- Hauptwurzel ohne Krankneitssymptome am Tag 5; Spur 11- Hauptwurzel ohne Krankheitssymptome am Tag 6; Spur 12- nichtinfizierter Keimling; Spur 13- P. citricola-DNA; Spur 14- PCR ohne DNA.

## Patentansprüche

1. Primer zum artspezifischen Nachweis von Phytophthora-Arten,
dadurch gekennzeichnet, daß
die Oligonukleotidfolge des Primers ausgewählt ist aus 5*'*- TCT TGC TTT TTT TGC GAG CC-3*'* (CITR1) oder 5*'*- CGC ACC GAG GTG CAC ACA AA-3*'* (CITR2) zum Nachweis von P. citricola,
die Oligonukleotidfolge des Primers ausgewählt ist aus 5*'*- GTG ACG TAG GTT CAT CTG CT-3*'* (CAMB3) oder 5*'*-GTG ACG TAG GCC AAA ATA ACA-3*'* (CAMB4) zum Nachweis von P. cambivora, und
die Oligonukleotidfolge des Primers ausgewählt ist aus 5*'*- GTG ATC GCA GGA GTG CTC TT-3*'* (QUERC1) oder 5*'*-GTG ATC GCA GTA AGA AAT GAG T-3*'* (QUERC2) zum Nachweis von P. quercina.

2. Primer nach Anspruch 1,
dadurch gekennzeichnet, daß
solche Primer-Sequenzen verwendet werden, die durch Addition, Deletion, Substitution und/oder chemische Modifikation zumindest eines Nukleotids verändert wurden, wobei die artspezifische Bindung an genomische DNA von Phytophthora citricola, Phytophthora cambivora oder Phytophthora quercina beibehalten wird.

3. Primer nach Anspruch 1 oder 2,
dadurch gekennzeichnet, daß
solche Primer-Sequenzen eingesetzt werden, deren Basenfolge komplementär zu den angegebenen Oligonukleotidprimersequenzen ist und/oder die eine Homologie von ≥90 % zu den Primer-Sequenzen aufweisen und/oder die unter stringenten Bedingungen mit den Primer-Sequenzen hybridisieren, wobei die artspezifische Bindung an genomische DNA von P. citricola, P. cambivora oder P. quercina beibehalten wird.

4. Primer nach nach einem oder mehreren der vorhergehenden Ansprüche,
dadurch gekennzeichnet, daß
die abgeleiteten RNA-Sequenzen der angegebenen Desoxyribonukleotidprimer eingesetzt werden.

5. Verfahren zum Nachweis von Phytophthora-Arten,
dadurch gekennzeichnet, daß
die DNA, die auf das Vorliegen von Phytophthora-Arten zu untersuchen ist, entweder
a) in einer Polymerase-Kettenreaktion (PCR) in Kontakt gebracht wird mit einem der nachfolgenden Oligonukleotid-Primerpaare, wobei
- das Primerpaar CITR1/CITR2 (5*'* -TCT TGC TTT TTT TGC GAG CC-3*'* und 5*'*-CGC ACC GAG GTG CAC ACA AA-3*'*) spezifisch an genomische DNA von Phytophthora citricola bindet;
- das Primerpaar CAMB3/CAMB4 (5*'*-GTG ACG TAG GTT CAT CTG CT-3*'* und 5*'*-GTG ACG TAG GCC AAA ATA ACA-3*'*) spezifisch an genomische DNA von Phytophthora cambivora bindet;
- und das Primerpaar QUERC1/QUERC2 (5*'*-GTG ATC GCA GGA GTG CTC TT-3*'* und 5*'*-GTG ATC GCA GTA AGA AAT GAG T-3*'*) spezifisch an genomische DNA von Phytophthora quercina bindet, und
im Verlauf der PCR-Amplifikation durch Verlängerung der Primersequenzen ein charakteristisches spezies-spezifisches DNA-Markerfragment entsteht; und
das spezies-spezifische DNA-Markerfragment nachgewiesen wird;
oder
b) unter stringenten Hybridisierungsbedingungen die Oligonukleotid-Primer CITR1, CITR2, CAMB3, CAMB4, QUERC1 und QUERC2 jeweils einzeln zur Hybridisierung gebracht werden und das Produkt dieser stringenten Hybridisierungsreaktion nachgewiesen wird.

6. Verfahren nach Anspruch 5,
dadurch gekennzeichnet, daß
solche Primer-Sequenzen verwendet werden, die durch Addition, Deletion, Substitution und/oder chemische Modifikation zumindestens eines Nukleotids verändert wurden, wobei die artspezifische Bindung an genomische DNA von Phytophthora citricola, Phytophthora cambivora oder Phytophthora quercina beibehalten wird.

7. Verfahren nach Anspruch 5 oder 6,
dadurch gekennzeichnet, daß
solche Primer-Sequenzen eingesetzt werden, deren Basenfolge komplementär zu den angegebenen Oligonukleotidprimersequenzen ist und/oder die eine Homologie von ≥ 90 % zu den Primer-Sequenzen aufweisen und/oder die unter stringenten Bedingungen mit den Primer-Sequenzen hybridisieren, sodaß die artspezifische Bindung an genomische DNA von P. citricola, P. cambivora oder P. quercina beibehalten wird.

8. Verfahren nach Anspruch 5, 6 oder 7,
dadurch gekennzeichnet, daß
die abgeleiteten RNA-Sequenzen der angegebenen Desoxyribonukleotidprimer eingesetzt werden.

9. Verfahren nach einem oder mehreren der vorhergehenden Ansprüche,
dadurch gekennzeichnet, daß
die DNA in isolierter Form eingesetzt wird oder im biologischen Material in für die Hybridisierungsreaktion zugänglicher Form vorliegend eingesetzt wird.

10. Verfahren nach einem oder mehreren der vorhergehenden Ansprüche,
dadurch gekennzeichnet, daß
die Primerpaare CITR1/CITR2, CAMB3/CAMB4 und QUERC1/QUERC2 nach einem oder mehreren der vorhergehenden Ansprüche in einer art-spezifischen PCR-Reaktionen verwendet werden, wobei
(a) die zu untersuchende DNA denaturiert wird, um Einzelstränge zu erhalten;
(b) die Primer-Paare an die beiden komplementären Einzel-stränge der zu untersuchenden DNA gebunden werden;
(c) die einzelnen Primer mittels einer DNA-Polymerase verlängert werden; und
(d) die Hitze-Denaturierungs-Primer-Bindungs- und Verlängerungszyklen wiederholt werden, um eine nachweisbare Menge des PCR-Produkts zu erhalten; und
(e) das erhaltene PCR-Produkt nachgewiesen wird.

11. Verfahren nach einem oder mehreren der vorhergehenden Ansprüche 5 bis 9,
dadurch gekennzeichnet, daß
bei der Alternative (b) eine Hybridisierungstemperatur eingesetzt wird, die 5-10°C unter dem jeweiligen Primerschmelzpunkt liegt.

12. Verwendung eines Primer-Oligonukleotids nach einem oder mehreren der vorhergehenden Ansprüche 1 bis 4,
dadurch gekennzeichnet, daß
die Oligonukleotid-Primer einzeln in einer Hybridisierungs-Reaktion oder paarweise in einer PCR-Reaktion zum spezifischen Nachweis und/oder zur Unterscheidung von anderen Phytophthora-Arten und/oder zur Quantifizierung von Phytophthora citricola-DNA, Phytopthora cambivora-DNA oder Phytophthora quercina-DNA eingesetzt werden.

13. Test-Kit, enthaltend zumindest einen Oligonukleotid-Primer nach einem oder mehreren der Ansprüche 1 bis 4 in einem geeigneten Behältnis.

14. Kit, nach Anspruch 13,
enthaltend ein Primer-Paar nach einem oder mehreren der Ansprüche 1 bis 4 zum Primen der Synthese der komplementären DNA in einer PCR-Reaktion.


SEQUENZPROTOKOLL

<110> GSF-Forschungszentrum für Umwelt und Gesundheit

<120> Nachweisverfahren für Phytophthora-Arten

<130> P11132

<140> DE 198 15 138.1
<141> 1999-06-30

<160> 10

<170> PatentIn Ver. 2.1

<210> 1
<211> 20
<212> DNA
<213> Phytophthora cambivora

<400> 1
gtgacgtagg ttcatctgct                                                    20

<210> 2
<211> 21
<212> DNA
<213> Phytophthora cambivora

<400> 2
gtgacgtagg ccaaaataac a                                                  21

<210> 3
<211> 20
<212> DNA
<213> Phytophthora citricola

<400> 3
tcttgctttt tttgcgagcc                                                    20

<210> 4
<211> 20
<212> DNA
<213> Phytophthora citricola

<400> 4

cgcaccgagg tgcacacaaa                                                    20

<210> 5
<211> 19
<212> DNA
<213> Phytophthora sp.

<400> 5
tccgtaggtg aacctgcgg                                                     19

<210> 6
<211> 20
<212> DNA
<213> Phytophthora sp.

<400> 6
tcctccgctt attgatatgc                                                    20

<210> 7
<211> 10
<212> DNA
<213> Phytophthora quercina

<400> 7
gtgatcgcag                                                               10

<210> 8
<211> 10
<212> DNA
<213> Phytophthora cambivora

<400> 8
gtgacgtagg                                                               10

<210> 9
<211> 20
<212> DNA
<213> Phytophthora quercina

<400> 9
gtgatcgcag gagtgctctt                                                    20

<210> 10
<211> 22
<212> DNA
<213> Phytophthora quercina

<400> 10
gtgatcgcag taagaaatga gt 22